(19) **Europäisches Patentamt / European Patent Office / Office européen des brevets**

(11) **EP 4 082 635 A1**

(12) **EUROPEAN PATENT APPLICATION**
published in accordance with Art. 153(4) EPC

(43) Date of publication:
**02.11.2022 Bulletin 2022/44**

(21) Application number: **20906820.4**

(22) Date of filing: **24.12.2020**

(51) International Patent Classification (IPC):
*A63B 22/02* (2006.01)    *A63B 22/06* (2006.01)
*A63B 23/04* (2006.01)    *A63B 24/00* (2006.01)
*A63B 69/00* (2006.01)    *A63B 71/06* (2006.01)

(52) Cooperative Patent Classification (CPC):
**A63B 22/02; A63B 24/00; A63B 69/00;
A63B 71/06;** A63B 22/06; A63B 23/04

(86) International application number:
**PCT/JP2020/048346**

(87) International publication number:
**WO 2021/132426 (01.07.2021 Gazette 2021/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.12.2019 JP 2019236645**

(71) Applicants:
• **The University of Tokyo
Bunkyo-ku, Tokyo 113-8654 (JP)**
• **Ohtake Root Kogyo Co., Ltd.
Ichinoseki-shi, Iwate 021-0902 (JP)**

(72) Inventors:
• **NAKAMURA, Yoshihiko
Tokyo 113-8654 (JP)**
• **IKEGAMI, Yosuke
Tokyo 113-8654 (JP)**
• **OTA, Yoshitake
Ichinoseki-shi, Iwate 021-0902 (JP)**
• **KANZAKI, Yuji
Sendai-shi, Miyagi 981-0933 (JP)**
• **KOGA, Hirotaka
Tokyo 113-8654 (JP)**

(74) Representative: **Plougmann Vingtoft a/s
Strandvejen 70
2900 Hellerup (DK)**

(54) **SMART TREADMILL**

(57)    A treadmill according to the present invention includes: a frame; an endless belt; an endless-belt drive unit; a plurality of cameras provided on the frame such that the orientations and/or locations thereof can be adjusted; a motion-data acquisition unit that markerlessly acquires motion data of a subject by using image information obtained with camera images; a motion analysis unit that analyzes the motion of the subject by using the motion data; and a control unit that controls the endless-belt drive unit based on the motion data.

FIG.7

## Description

## Technical Field

The present invention relates to a treadmill, and more particularly to a smart treadmill having a motion capture ability and a motion analysis ability.

## Background of the Invention

A treadmill is a device widely used in exercise training and rehabilitation. Some treadmills are equipped with a force plate or a position detection sensor, and some treadmills control the speed of the treadmill according to the detection result (Patent Documents 1 to 9), but the treadmill is basically provided as a product with a single function (walking/running function). Therefore, in the case of analyzing and evaluating the walking motion of the subject on the treadmill, the motion of the subject is measured by placing the treadmill in a motion capturing space.

As a typical motion capture technique, optical motion capture and motion capture using inertial sensors are known. According to the optical motion capture, a plurality of optical markers coated with a retroreflective material are attached to the body of a subject, a plurality of cameras such as an infrared camera take images of the motion of the subject, and the motion of the subject is obtained from the movement trajectory of the optical markers. In the optical motion capture using an infrared light source, it is necessary to perform measurement in a space surrounded by cameras that is not affected by external light, and the measurement space is restricted.

For both the optical motion capture and motion capture using inertial sensors, it is necessary to attach multiple markers or multiple sensors to the body of the subject, and there are drawbacks in which the preparation for motion capture requires time and manpower, and natural motion may be hindered by constraint of the movement of the subject.

Recently, so-called marker-less motion capture technology has appeared. In the present specification, the term "marker-less" means not using markers or sensors attached to the body of a subject. As a marker-less motion capture technique, a method of estimating a joint position from a distance image (depth image) or a method of estimating a joint position from an RGB image is known. For example, the video motion capture technology (Non-Patent Document 1) is a technique for performing motion capture from video images of a plurality of RGB cameras in a completely unconstrained manner.

## Citation List

[Patent Document]

[1] US PAT. NO. 4,830,021
[2] Japanese Patent Application Laid-Open No. 2013-2226340
[3] Japanese Patent Application Laid-Open No. 2015-107247
[4] US PAT. NO. 4,601065
[5] US PAT. NO. 8002672
[6] US PAT. NO. 6,173608
[7] Japanese Patent Application Laid-Open No. 10-243979
[8] US Pat. NO. 5,368,532
[9] US PAT. NO. 5,800,314

[Non-Patent Document]

[1] T. Ohashi, Y. Ikegami, K. Yamamoto, W. Takano and Y. Nakamura, Video Motion Capture from the Part Confidence Maps of Multi-Camera Images by Spatiotemporal Filtering Using the Human Skeletal Model, 2018 IEEE/RSJ International Conference on Intelligent Robots and Systems (IROS), Madrid, 2018, pp. 4226-4231.
[2] Open pose. https://github.com/CMU-Perceptual-Computing-Lab/openpose.
[3] http://cocodataset.org/#keypoints-leaderboard
[4] Y. Nakamura, K. Yamane, Y. Fujita, and I. Suzuki. Somatosensory computation for man-machine interface from motion-capture data and musculoskeletal human model. Trans. Rob., 21(1):58-66, Feb 2005.

## Summary of the Invention

The present invention seeks to provide a high functionality treadmill by combining a treadmill with marker-less motion capture.

The present invention relates to a treadmill integrated with motion capture comprising:

a frame;
an endless belt that provides a walking surface on which a subject stands;
a belt drive unit;
a plurarity of cameras provided on the frame in which orientations and/or locations of cameras are adjustable; and
a motion data acquisition unit that acquires motion data of the subject markerlessly using image information obtained from each camera image.

In one aspect, the treadmill comprises a motion analysis unit that analyzes the motion of the subject using the motion data.

In one aspect, the motion analysis unit analyzes the motion of the subject by using the operation information of the endless belt (velocity information or the incline of the belt) in addition to the motion data.

In one aspect, the treadmill comprises a control unit that controls the operation of the treadmill based on the motion data.

The operation of the treadmill includes the rotation of the endless belt and the incline of the endless belt. By controlling a rotary drive unit of the endless belt and an incline drive unit of the endless belt, it is possible to control the traveling speed (belt speed) of the endless belt and the incline of the endless belt.

In one aspect, the control unit comprises a data generation unit that generates input data based on the motion data; and a control signal generator that generates a control signal according to the input data, wherein the control unit outpus the control signal to the belt drive unit to control the belt speed.

In one aspect, the endless belt can be inclined by an incline drive unit, and the control unit outputs the control signal to the incline drive unit to control the incline of the belt.

In one aspect, the motion data acquisition unit obtains a spatial distribution of a likelihood of a position of each joint of the subject using image information of the subject obtained by each camara image, and obtains 3D pose of the subject at each frame based on the spatial distribution of the likelihood.

In one aspect, a distance between adjacent joints is obtained as a constant, the motion data acquisition unit is configured to:

obtains one or a plurality of position candidates corresponding to each feature point based on the spatial distribution of the likelihood;
obtains each joint angle by performing an optimization calculation based on inverse kinematics using the candidates and an articulated structure; and
obtains positions of the feature points including the joints by performing a forward kinematics calculation using the joint angles.

In one aspect, the spatial distribution of the likelihood is used in the optimization calculation based on inverse kinematics.

In one aspect, the processing unit sets a search range for feature point position candidates using each feature point obtained at one or a plurality of frames, and obtains one or a plurality of feature point position candidates for each joint position in a frame at time t+1 using the spatial distribution of the likelihood obtained in the frame at time t+1.

In one aspect, the search range is a set of a predetermined number of points three-dimensionally distributed at a predetermined interval around the position of the feature point.

In one aspect, the processing unit is configured to:

smooth the positions of feature points in a temporal direction using a plurality of positions of the feature points obtained at a plurality of the other frames; and
obtain each joint angle of the subject by performing the optimization calculation based on inverse kinematics using the smoothed positions of feature points and the articulated structure of the subject.

The treadmill according to the present invention can measure the motion of a subject walking or running on the treadmill by simply providing a plurality of video cameras and executing a predetermined algorithm using a computer. With a simpler configuration, motion measurement and motion analysis can be performed in real time, and there is no need to attach markers to the subject or prepare to provide an elaborate motion capturing space. The burden on both the subject wearing the markers and a physiotherapist is greatly reduced, and the motion measurement and motion analysis of the subject can be performed with almost the same preparation as when using a normal treadmill.

According to the present invention, by combining the treadmill with marker-less motion capture, it is possible to deal with any kinds of control of the treadmill based on the motion data acquired by one means, namely, motion capture.

By performing motion analysis or treadmill operation control based on motion data measured by motion capture, the work that was conventionally performed visually by a physiotherapist, for example, in walking training, can be dealt with by the treadmill ability.

**Brief Description of the Drawings**

FIG. 1 shows a perspective view of the treadmill (dual belt) according to the embodiment.

FIG. 1A shows a perspective view of the treadmill (single belt) according to the embodiment.

FIG. 2 shows a plan view and a side view of the treadmill shown in FIG. 1 and shows the first mode of camera arrangements.

FIG. 3 shows a top view and a side view of the treadmill and shows the second mode of camera arrangements.

FIG. 4 shows a top view and a side view of the treadmill and shows the third mode of camera arrangements.

FIG. 5 shows a first mounting structure of the camera posture.

FIG. 6 shows a second mounting structure of the camera posture.

FIG. 7 is a block diagram which shows the structure of the treadmill according to the embodiment.

FIG. 7A is a block diagram of the treadmill control system according to the embodiment.

FIG. 7B is a block diagram of the control system for a dual-belt treadmill.

FIG. 8 is an overall view of the motion data acquisition unit of the present embodiment.

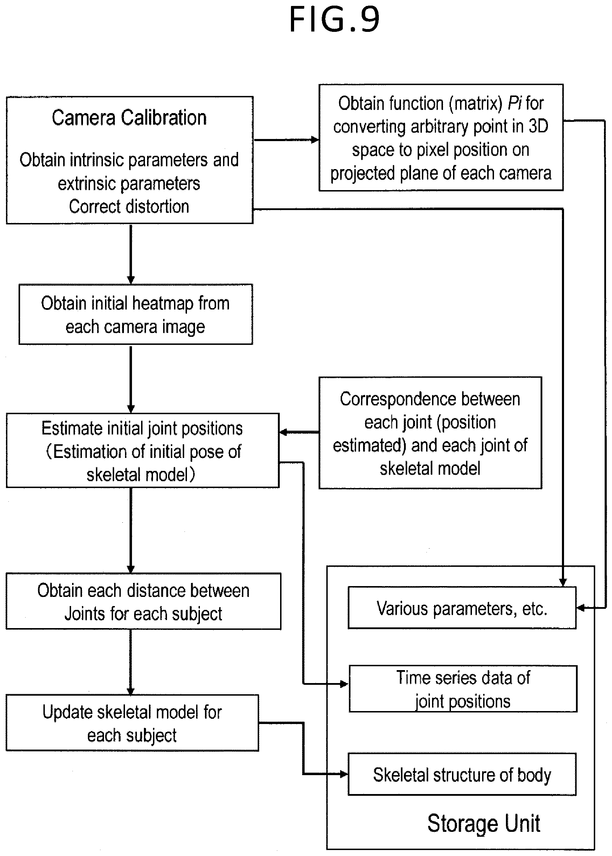

FIG. 9 is a flowchart showing processing steps for camera calibration and acquisition of an initial pose and a distance between joins of a skeleton model.

FIG. 10 shows a skeleton model of the present embodiment(a left figure) and keypoints provided by OpenPose (a right figure).

FIG. 11 is a flowchart showing processing steps performed by a unit for obtaining a joint position candidate.

FIG. 12 is a flowchart showing steps for motion analysis using motion capture.

FIG. 13 is a schematic which shows the camera calibration according to the embodiment.

FIG. 14 is a block diagram which shows the calculation method of the target belt speed in the operation control of the treadmill according to the embodiment.

**Description of the Embodiments**

[A] Overall Configuration of Smart Treadmill

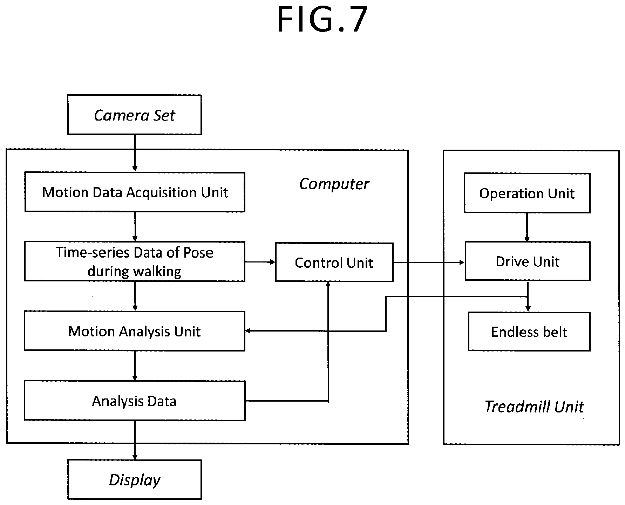

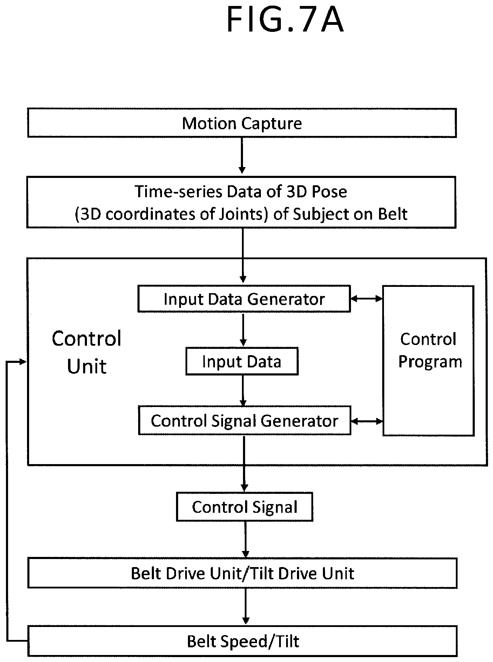

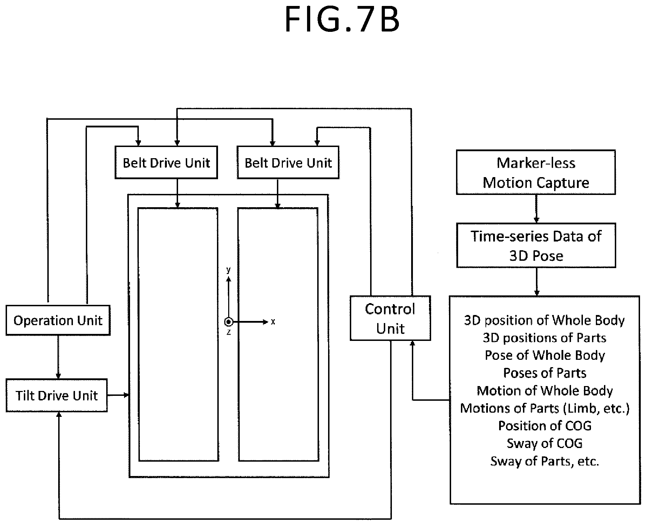

As shown in FIG. 7, a smart treadmill according to the present embodiment relates to a treadmill system which comprises a treadmill, a plurality of cameras, one or a plurality of computers, and a display. The treadmill comprises an endless belt that provides a walking surface on which a subject stands, a drive unit, and an operation unit. The smart treadmill further comprises a motion data acquisition unit, a motion analysis unit, and a control unit, which are constituted by a computer. The motion data acquisition unit comprises, for example, a markerless motion capture system, and the motion data acquisition unit acquires time-series data of a 3D pose during an exercise of the subject (for example, during walking). The motion analysis unit outputs analysis data by processing motion data. The control unit outputs a predetermined instruction to the drive unit of the treadmill to control the operation of the treadmill using the motion data and/or the analysis data.

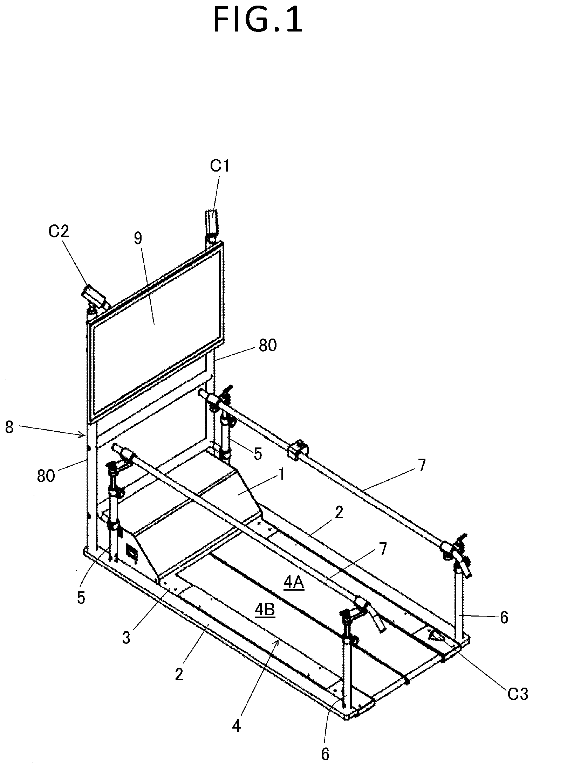

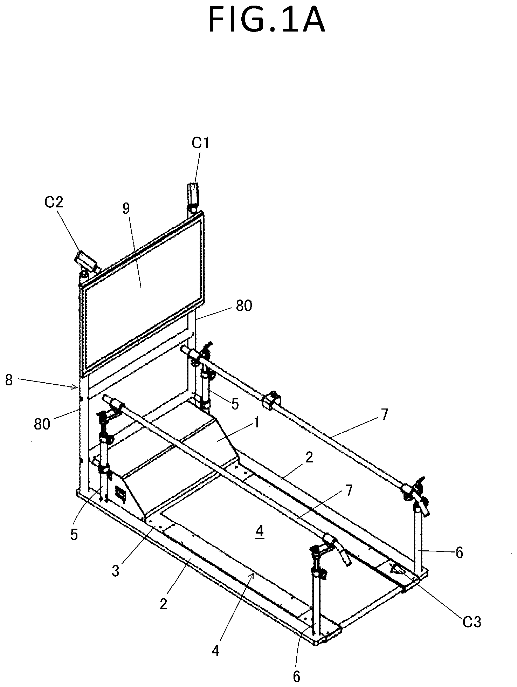

As shown in FIGS. 1 to 4, the treadmill comprises a base frame having a front base 1 and left and right side frames 2, a tread base 3 supported between the left and right side frames 2, and an endless belt (running belt) that runs above and below the tread base 3. The endless belt 4 is wound around a front roller (drive roller) rotatably provided at a forward portion of the base frame and a rear roller (driven roller) rotatably provided at the base frame. An upper portion of the endless belt 4 forms a walking surface or a running surface (hereinafter, collectively referred to as a "walking surface").

In the embodiments shown in FIGS. 1, 2, 3 and 4, the endless belt 4 comprises two endless belts (a first belt 4A for the right foot and a second belt 4B for the left foot) running in parallel. The treadmill comprises a first motor (not shown) for driving the first belt 4A and a second motor (not shown) for driving the second belt 4B. Such a dual-belt treadmill is known and is disclosed in, for example, Patent Documents 2, 6 and 7. For a specific configuration, Patent Documents 2 and 7 can be referred to. As shown in FIG. 1A, the endless belt 4 may be a single belt.

The treadmill comprises a belt drive unit including a motor located inside the front base 1, and the endless belt 4 is caused to run by driving the drive rollers with the belt drive unit. The rotation speed of the motor is variable, and the speed of the endless belt 4 can be adjusted by changing the rotation speed of the motor. An adjustment of the speed of the endless belt 4 can be operated from an operation unit (not shown). An operation unit is provided with, for example, a start button, a stop button, an acceleration button, and a deceleration button. The operation unit may be provided with an incline adjustment button for inclining the walking surface of the endless belt 4. An adjustment of the incline of the endless belt 4 is performed by, for example, the incline mechanism of the tread base 3 and the endless belt 4. It can be adjusted by operating an actuator (motor, hydraulic pressure, air pressure, etc.) of the incline mechanism by an instruction from the incline adjustment button to change the incline of the tread base 3. An incline direction of the belt by the incline mechanism is typically a longitudinal incline (the heights of the front and rear ends of the belt are different), but in addition to or instead of, the incline direction may be a lateral incline (the heights of the left and right ends of the belt are different). The speed of the endless belt 4 can be controlled by the control unit. Further, the incline of the endless belt 4 may be controlled by the control unit.

First support columns 5 are provided on the forward side of the left and right side frames 2, and second support columns 6 are provided on the rear side of the left and right side frames 2. A handrail frame 7 is provided between an upper end of the first support and an upper end of the second support. A front frame 8 including left and right columns 80 is erected at the forward end of the base frame, and a display 9 is supported by the front frame 8. In the present specification, in the treadmill, portions other than the endless belt 4, or a structural bodies (the front base 1, the side frame 2, the tread base 3, the first support columns 5, the second support columns 6, the handrail frames 7, the front frame 8) are collectively called a frame.

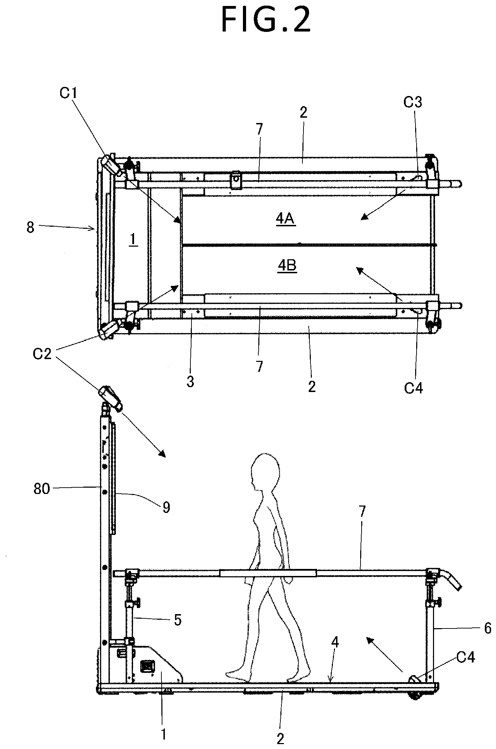

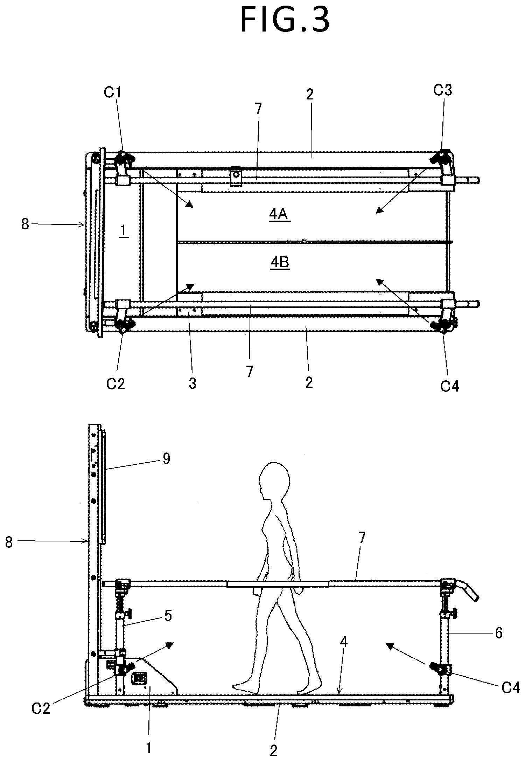

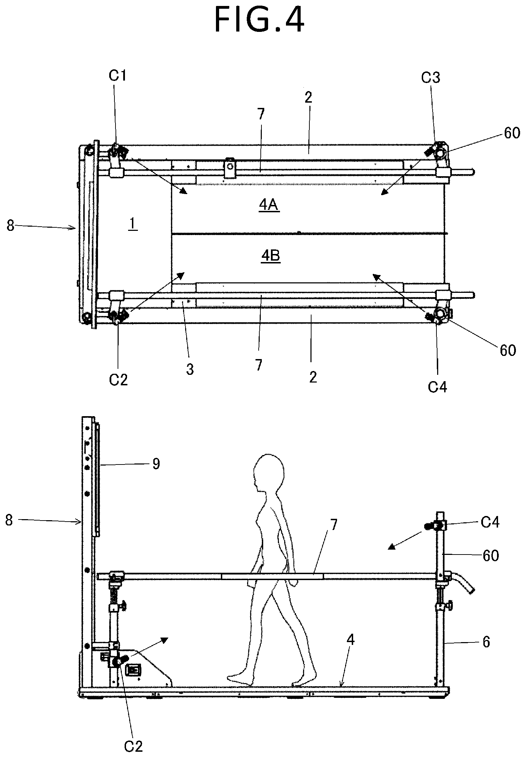

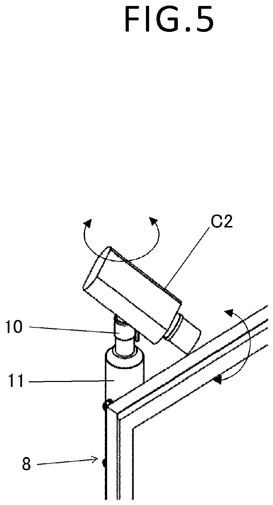

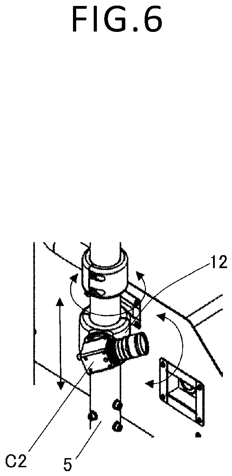

The treadmill according to the present embodiment comprises four cameras C which are arranged so as to surround the walking surface, and a subject on the running belt is photographed by four cameras having different viewpoints. In the embodiment shown in FIGS. 1 and 2, cameras C1 and C2 are provided at upper ends of the left and right columns 80 of the front frame 8, and cameras C3 and C4 are provided at rear ends of the left and right side frames 2 or the widthwise sides of the rear end of the tread base 3. In the embodiment shown in FIGS. 3A and 3B, cameras C1 and C2 are provided on the left and right first columns 5, and cameras C3 and C4 are provided on the left and right second columns 6. In the embodiment shown in FIGs. 4A and 4B, the left and right second columns 6 are provided with extending portions 60 extending upward beyond the handrail bar 7, and cameras C1 and C2 are provided on the left and right first columns 5, and cameras C3 and C4 are provided on the extending portions 60 of the left and right second columns 6. The number and arrangement of the cameras shown in FIGS. 1 to 4 are examples, and are not limited to the illustrated modes. The cameras C1 to C4 are fixed to the frame in which the poses and/or the locations thereof can be adjusted. FIGS. 5 and 6 show examples of a camera mounting structure. The mounting structures of the camera shown in FIGS. 5 and 6 are illustrative, and the mounting structure of the camera is not limited to the illustrated embodiments.

According to the first embodiment, the cameras C1 and C2 (FIGS. 1 and 2) are attached to the upper end of the front frame 8 in which the pose of camera is adjustable. As shown in FIG. 5, the camera C2 is vertically rotatably attached to an upper end of a first axis 10 in which the first axis 10 is rotatably provided in a cylindrical second axis fixed to the frame 8. The height positions of the cameras C1 and C2 may be adjustable with respect to the frame 8. According to the first embodiment, the cameras C3 and C4 (FIGS.1 and 2) may be configured to be stowable in the side frame 2 or the tread base 3 (for example, when stored, they are flush with an upper surface of the frame).

According to the second and the third embodiments, the cameras C1 and C2 (FIGS. 3 and 4) are attached to the first support columns 5 in which the pose and height position of the camera can be adjusted. As shown in FIG. 6, the camera C2 is vertically rotatably attached to the peripheral surface of a tubular body 12, and the tubular body 12 is attached to the first support column 6 in which the tubular body 12 is rotatable around the circumferential direction around the outer periphery of the first support column 6 and elevationally moveable along the first support column 6. A structure similar to the above mentioned mounting structure of the cameras C1 and C2 can be adopted for the mounting structure of the cameras C3 and C4 (FIGS. 3 and 4) according to the second and third embodiments.

When the endless belt 4 of the treadmill can be inclined, the cameras are attached to an immovable portion that is not affected by the incline of the endless belt 4 during the measurement of the walking motion of the subject. For example, when the cameras are attached to any of the first support column 5, the second support column 6, the handrail frame 7, and the front frame 8, the pose and location of the camera remain unchanged even if the endless belt 4 and the tread base 3 are inclined.

The plurality of cameras C1 to C4 are attached to the frame with their poses and locations adjusted so that the whole body of the subject on the walking surface of the treadmill can be photographed. In the illustrated embodiment, four cameras C1 to C4 are shown, but the number of cameras is not limited. Further, a mirror may be provided to reduce the number of cameras. The camera may include a wide-angle lens or a fisheye lens. When the wide-angle lens or the fisheye lens is used, image distortion correction is appropriately applied, and a distrotion-corrected image is used. Since the plurality of cameras C1 to C4 are incorporated in the frame of the treadmill, motion capture can be performed in substantially the same space as the space occupied by the treadmill.

A computer comprises a processor that processes data and a memory that stores data. The computer functions as a motion data acquisition unit that acquires the motion data of the subject (time series data of 3D pose) from the image data of the subject, a storage unit for the time series data of the subject obtained by the motion data acquisition unit, a motion analysis unit using the time series data of the subject, a storage unit for analysis data by the motion analysis unit, and a control unit for controlling the treadmill based on the analysis data. In one aspect, the computer is installed in a frame, for example, a front base 1. Further, the computer may comprise a local computer provided in the frame and a computer capable of wireless data communication between the local computer or a could-based computer. In one embodiment, a computer for motion capture, a computer for motion analysis, and a computer for controlling a treadmill are separately prepared.

The treadmill according to the present embodiment may be provided with a force plate located below the upper portion of the endless belt 4. A treadmills provided with force plates is known and disclosed, for example, in Patent Documents 1-6. Patent Documents 1 to 3 disclose a treadmill including a force plate using a strain gauge. Patent Documents 4 and 5 disclose a treadmill including a force plate using a capacitance type sensor. Patent Document 6 discloses a treadmill including a crystal oscillator force sensor capable of detecting in three directions. The time-series data acquired by the force plate when the subject walks or runs on the running belt is stored in the storage unit of the computer. The data acquired by the force plate can be used for analyzing motion by the motion analysis unit. Further, the treadmill may be provided with a non-weight bearing device.

[B] Marker-less Motion Capture

[B-1] Overall Configuration of Marker-less Motion Capture

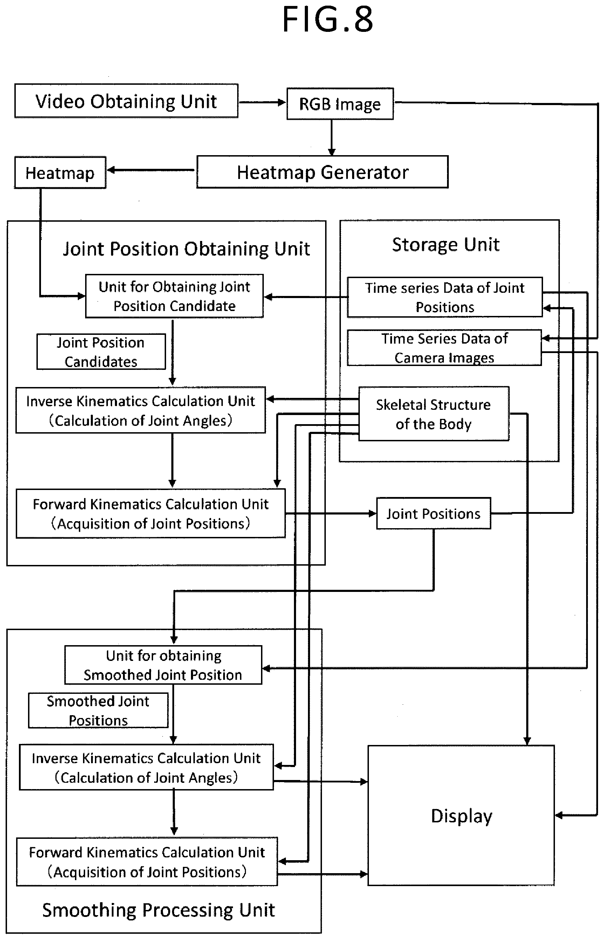

A marker-less motion capture system according to the present embodiment comprises a plurality of video cameras mounted on a frame of a treadmill and a computer. As shown in FIG.8, a motion capture system of the present embodiment comprises a video obtaining unit (video camera) for obtaining a motion of a subject, a heatmap generating unit for obtaining heatmap information presenting the degree of likelihood of a position for each feature point (keypoint) including joints with color intensities based on the images obtained by the video obtaining unit, a unit for obtaining joint positions of the subject by using the heatmap information obtained at the heatmap generating unit, a smoothing processing unit for smoothing the joint positions obtained by the unit for obtaining joint positions, a storage unit for storing a skeletal structure of the body of the subject, time series data of images obtained at the video obtaining unit, time series data of the joint positions obtained at the unit for obtaining joint positions, and so forth, and a display for displaying the image of the subject captured by the video obtaining unit, the skeletal structure corresponding to the pose of the subject, and so forth. Since a feature point in the body of the subject is primarily a joint, the word "joint" is used in the description and drawings for representing a feature point, and it is noted that description to "joint" can be applied to a feature point other than the joints.

The video obtaining unit comprises a plurality of synchronized cameras, each camera images taken at the same time is sent to the heatmap generating unit, and the heatmap generating unit generates a heatmap for each camera image. The heatmap represents a spatial distribution of a likelihood of a position of a feature point in the body. The generated heatmap information is sent to the unit for obtaining joint positions and then joint positions are obtained by the unit for obtaining joint positions. The obtained joint position data is stored in a storage unit as time series data of the joint positions. The obtained joint position data is sent to the smoothing processing unit to obtain smoothed joint positions and joint angles. A pose of the subject can be determined by the smoothed joint positions, joint angles and the skeletal structure of the body of the subject, and the motion of the subject comprised of time series data of the pose may be displayed on the display.

The subject exercising on the walking surface of the treadmill comprises a link structure or an articulated structure. Typically, the subject is human, and the articulated structure is a skeletal structure of the body. It may be necessary to prepare learning date used for the heatmap generating unit corresponding to a subject, but a subject other than human (such as an animal other than human) is applicable. In addition, a subject is not limited to the whole body of the human but may be a part of the body.

A storage unit stores acquired data and processed data. For example, the storage unit stores time series data of the images taken by the video obtaining unit, and joint position data and joint angle data obtained by the unit for obtaining joint position. The storage unit may further store smoothed joint position data and smoothed joint angle data obtained by the smoothing processing unit, heatmap data generated by the heatmap generating unit and any other data generated during processing.

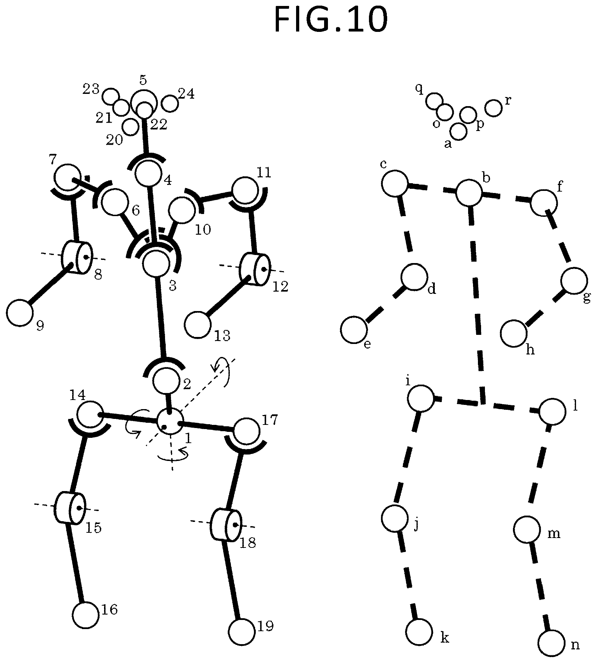

The storage unit further stores data determining the skeletal structure of the body of the subject. The data comprises a file for defining a skeletal mode of the body and distance data between the adjacent joints of the subject. The joint angles and the pose of the subject are determined by the joint positions in the skeletal model, which is an articulated object. The skeletal model used in the present embodiment is shown in the left figure of FIG.10. The skeletal model shown in the left figure of FIG.10 has 40 degrees of freedom, but this skeletal mode is illustrative. As will be described later, a constant representing the distance between adjacent joints of the subject can be obtained at the time of initial setting of motion capture. The distance between the adjacent joints of the subject may be obtained prior to motion capture by another method, or the already acquired distance may be used. According to the present embodiment, by using the skeletal structure data of the subject body, it is possible to give a constraint condition unique to the skeletal structure in calculating the joint positions in which the distance between adjacent joints does not change with time.

The display displays the motion video of the subject obtained by the video obtaining unit, time series skeletal images showing the pose of the subject obtained by motion capture and such. For example, skeletal image (pose of subject) data is generated in each frame by the processor of the computer by using a subject-dependent skeletal structure and obtained time series data of the joint angles and joint positions, and the skeletal image data is outputted at a predetermined frame rate to the display to show the motion video.

[B-2] Heatmap Generating Unit

A heatmap generating unit generates a two-dimensional or three-dimensional spatial distribution of a likelihood a position of a feature point (keypoint) in the body including joints based on an input image and represents the spatial distribution of the likelihood in a heatmap manner. In the embodiment, the likelihood obtained by the heatmap generating unit is given to each pixel on a 2D image, and the heatmap information from a plurality of viewpoints is integrated to obtain information on the probability of three-dimensionally existing position of the feature points. The heatmap shows varying values spreading over a space by color intensities in the space like a temperature distribution and enables visualization of the likelihood. In the present embodiment, it is sufficient that the heatmap generating unit obtains the spatial distribution of the likelihood of the positions of the feature points in the body including the joints, or heatmap information(each pixel of the image having a value representing the likelihood), and it is not necessary to display the heatmap.

The heatmap generating unit estimates the position of the feature point (typically joint position) on the subject body from a single input image as a heatmap by typically using a convolutional neural network (CNN). The convolutional neural network (CNN) comprises an input layer, an intermediate layer (a hidden layer) and an output layer and the intermediate layer is configured by deep learning using training data for two-dimensional mapping of a feature point to an existing position on an image.

As the heatmap generating unit, for example, OpenPose (Non-Patent Document 2), which is an open source software, can be used. According to OpenPose, 18 feature points (keypoints) in the body are set (see the right figure of FIG.10). Specifically, the 18 feature points consist of 13 joints, a nose, left and right eyes, and left and right ears. OpenPose uses a trained convolutional neural network (CNN) to generate Part Confidence Maps (PCM) of 18 featur points (keypoints) in the body from a single RGB image offline or in realtime to display PCM in a heatmap format. In the present specification, the term PCM may be used occasionally for a spatial distribution of a likelihood of a position of a feature point or a heatmap, however, it should be noted that an index representing the spatial distribution of the likelihood of the position of feature point in the body including each joint is not limited to PCM.

OpenPose is applied to each RGB image obtained from a plurality of synchronized cameras to generates Part Confidence Maps (PCM) for 18 feature points. A method other than OpenPose can be used for the heatmap generating unit. Various methods have been proposed as techniques for generating a heatmap showing the probability of the position of a feature point on the subject body. For example, a method that has won a high ranking in the COCO Keypoints challenge (Non-Patent Document 3) can be adopted. Alternatively, a learning device for the heatmap generating unit may be created independently, for example by using images of a subject taken by cameras mounted on a treadmill as learning data, to construct a convolutional neural network (CNN).

Here, it may be possible to acquire the 3D pose of the subject at each time by estimating the two-dimensional coordinates of the joint position (the coordinates of the centroid of the heatmap) using the heatmap obtained from each camera image and three-dimensionally reconstructing them . The present invention does not exclude such a motion data acquisition method but it is difficult to perfom motion measurement with high accuracy as with the optical motion capture technique due to erroneous estimation of two-dimensional coordinates of joint positions and such. The present embodiment adopts a method of three-dimensionally reconstructing the joint position using the heatmap information which can provide motion measurement with a high accuracy similar to that with the optical motion capture technique.

[B-3] Initial Setting of Motion Capture

Referring to FIG.3, camera calibration, acquisition of initial pose of the skeletal model, and acquisition of a distance between joints of the subject for the motion capture system of the present embodiment will be explained.

[B-3-1] Camera Calibration

To perform the motion capture using a plurality of cameras, camera parameters for three-dimensionally reconstructing a plurality of camera images are acuquired. The camera parameters include intrinsic parameters such as a focal length distance and an optic center, extrinsic parameters that represent the pose and position of the camera, and distortion parameters. Camera calibration for acquiring camera parameters may be conducted, for example, by placing a calibration device (a checker board, a calibration wand, etc.) having a known shape and dimensions in a space above the walking surface of the treadmill, taking pictures of the device by a plurality of cameras fixed to the frame with their poses and positions being adjusted, and peforming optimization calculation using each camera image. The distortion parameters can be obtained at the same time as the intrinsic parameters. Camera parameters can be used to obtain a projection matrix for projecting an arbitrary point in three-dimensional space onto the image plane of each camera. Using the projection matrix for each camera, the function (matrix) for converting the points of any three-dimensional shape to the pixel position of the projected plane of the camera Pi is obtained. The function (matrix) Pi is stored in the storage unit.

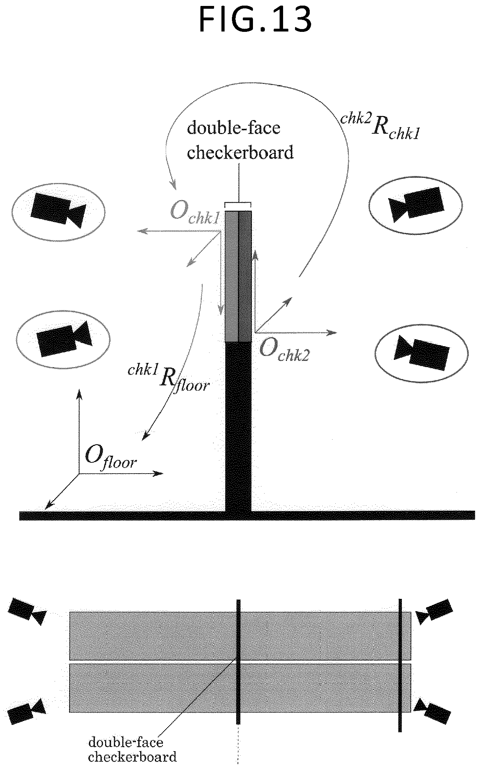

In one embodiment, calibration is performed using a double-face checkerboard with checker patterns on both sides. As shown in FIG. 13, the checkerboard is installed vertically on the walking surface (belt) of the treadmill. The checkerboard is provided, for example, in a vertical posture on a support frame erected located in the middle of the walking surface (belt) in the front-rear direction. The first checker pattern on the first surface of the checkerboard and the second checker pattern on the second surface match to each other. The relative position/orientation between the first checker pattern and the second checker pattern is determined. The first checker pattern is photographed by the two front cameras, and the second checker pattern is photographed by the two rear cameras. In one embodiment, the four cameras are mounted on the frame of the treadmill, but the four cameras may be independent of the treadmill. The four cameras are arranged two each for coordinate spaces $O_{chk1}$ and $O_{chk2}$ formed by the first checker pattern and the second checker pattern, respectively. Using the homogeneous transformation matrix $^{chk2}R_{chk1}$ determined by the relative position/orientation between the first checker pattern and the second checker pattern, a point represented as $p_{chk2}$ in the coordinate space $O_{chk2}$ can be expressed as a coordinate $p_{chk1}$ in the coordinate space $O_{chk1}$ by the following equation.

$$\boldsymbol{p}_{chk1} = \left(^{chk2}\boldsymbol{R}_{chk1}\right)^{-1}\boldsymbol{p}_{chk2}$$

By this conversion, the relative positions and orientations of the four cameras can be expressed and acquired in the same space $O_{chk1}$. Further, based on the height at which the checker patterns on the checker board are provided and the flat plate being installed perpendicular to the floor, it is possible to derive a homologous transformation matrix $^{chk1}R_{floor}$ from the coordinate space $O_{chk1}$ to the world coordinate system $O_{floor}$ set on the floor. Therefore, the coordinates $p_{floor}$ in the world coordinate system $O_{floor}$ corresponding to a point expressed as $p_{chk1}$ in the coordinate space Ochk1, can be expressed by the following equation.

$$\boldsymbol{p}_{floor} = \left(^{chk1}\boldsymbol{R}_{floor}\right)^{-1}\boldsymbol{p}_{chk1}$$

From the above, through the coordinate spaces $O_{chk1}$ and $O_{chk2}$ , the coordinates of all points in the space and the positions and orientations of all the cameras can be represented by the world coordinate system $O_{floor}$. In the present embodiment, as shown in FIG. 13, the three-dimensional coordinates are defined by a coordinate space having an origin at the center of the walking surface of the treadmill, a y-axis in the direction from the rear to the front along the traveling direction of the treadmill belt, an x-axis in the widthwise direction of the belt, and a z-axis in the vertical upward direction. A position and an orientation of the treadmill belt and a position and a pose of the subject on the belt can be defined in world coordinates. The three-dimensional joint position data of the subject defines not only the pose of the subject at a certain time point but also the positional information of the subject on the belt. The speed of the belt is the speed along the y-axis. To summarize the calibration device according to the present embodiment, the plurality of cameras used for motion capture include one or a plurality of front cameras located on the front side of the endless belt and rear cameras located on the rear side of the endless belt. A calibration device can be installed in the treadmill, and the calibration device comprises a checkerboard located in the middle of the endless belt in the front-rear direction and provided vertically. A first checker pattern to be photographed by the front camera is provided on the first surface of the checkerboard, and a second checker pattern to be photographed by the rear camera is provided on the second surface of the checkerboard. FIG. 13 shows a mode in which motion capture is performed using two cameras on the front side and two cameras on the rear side. However, when motion capture is performed by only the two cameras on the front side or two cameras on the rear side, for example, the checker pattern on either side may be used. Further, the checkerboard in a horizontal posture installed parallel to the belt plane may be used alone or in combination with the double face checkerboard for calibration. Further, the double face checkerboard may be provided on the support frame so as to be rotatable between the vertical posture and the horizontal posture.

[B-3-2]Correspondence between Skeletal Model and Feature Points in body on which Heatmap is Generated

Each joint of the skeletal model (the left figure of FIG.10) corresponds to each feature point in the body adopted by the heatmap generating unit (the right fibure of FIG.10, except a, o, p, q, r). The correspondence is shown in table 1.

[Table1]

| Correspondence of Human Model to OpenPose | | | |
| --- | --- | --- | --- |
| 40DOF Human Model (Fig. 10 left) | | | OpenPose (Fig. 10 right) |
| Joint Number | Name | DOF | Reference |
| 1 | Pelvis (Base body) | 6 | - |
| 2 | Waist | 3 | - |
| 3 | Chest | 3 | - |
| 4 | Neck | 3 | b |
| 5 | Head | 3 | - |
| 6 | Right Clavicle | 3 | - |
| 7 | Right Shoulder | 3 | c |
| 8 | Right Elbow | 1 | d |
| 9 | Right Wrist | 0 | e |
| 10 | Left Clavicle | 3 | - |
| 11 | Left Shoulder | 3 | f |
| 12 | Left Elbow | 1 | g |
| 13 | Left Wrist | 0 | h |
| 14 | Right Hip | 3 | i |
| 15 | Right Knee | 1 | j |
| 16 | Right Ankle | 0 | k |
| 17 | Left Hip | 3 | l |
| 18 | Left. Knee | 1 | m |
| 19 | Left Ankle | 0 | n |

The joints of the skeletal mode of the present embodiment and 18 keypoints provided by OpenPose are not completely corresponded. For example, there are no keypoints provided by OpenPose which correspond to pelvis (base body), waist, chest, right clavicle, left clavicle, head of the skeletal model. Both the joints of the skeletal model of the present embodiment and 18 keypoints provided by OpenPose are typical feature points in the body and may not cover all possible feature points. Additional detail feature points may be provided. Alternatively, all of feature points of the body can be joints. Joint angles which cannot be determined with 18 keypoints provided by OpenPose can be determined by optimization considering a constraint such as a movable range. If the joints of the skeletal model and the feature points on which the spatial distribution of the likelihood is obtained correspond to each other from the beginning, such correspondence may not be necessary.

[B-3-3] Acquisition of Initial Pose and Distance between Joints of Skeletal Model

An initial pose is obtained as a starting point for motion measurement of a subject. According to the embodiment, estimations of initial pose of skeletal model and distance between adjacent joints are obtained from the pixel positions of feature points that are calculated by applying OpenPose to an initial image. For example, the pixel position of the centroid of the initial heatmap for each feature point calculated by OpenPose is estimated as the two-dimensional position of the feature point. An initial position of the feature point in the three-dimensional space is estimated using the positions of the corresponding points on each camera image and the camera parameters. If it is determined that the initial position is erroneous, the initial setting is performed again. For example, erroneous estimation is determined by setting a threshold value for the PCM score of each coordinate when the estimated 3D position of the feature point is projected on each camera image.

An optimization calculation based on inverse kinematics requires a constant of the distance between adjacent feature points (a distance between joints), or a link length. However, since the link length differs for each subject, the link length of the skeletal model is calculated for each subject. It is desirable to perform scaling for each subject to improve the accuracy of motion capture of the embodiment. The skeletal model is a model of a standard human skeletal structure, and a skeletal model adapted for the subject body is generated by scaling the whole body or each section.

In this embodiment, each link length of the skeleton model is updated based on the obtained initial pose. With respect to the initial link length of the skeleton model, the scaling parameters used for updating the link length are calculated from the positions of the feature points based on the correspondence shown in FIG.10 and Table 1. As for the link lengths in the left figure of FIG.10, the link lengths of 1-2, 2-3, 3-4, 3-6, 3-10, 6-7, and 10-11 do not have the corresponding keypoints and the scaling parameters cannot be determined by the similar way. Therefore, these link lengths are determined using scale parameters of the other links. In this embodiment, since the human skeleton basically has symmetrical lengths, each scaling parameter is obtained from the left and right averages so as to be even on the left and right, and the initial link length of the skeleton model is even on the left and right. Regarding the calculation of the scaling parameter between Neck and Head, the scaling parameter is calculated with the midpoint of the feature point position of both ears as the location of the head joint. Each link length of the skeleton model is updated using the obtained scaling parameters. Regarding the calculation of the positions of the nose, eyes, and ears, the positions of each virtual joint (nose, eyes, ears) are calculated based on the correspondence as shown in Table 2.

[Table2]

| Correspondence of Human Model to OpenPose | | | |
|---|---|---|---|
| 40DOF Human Model (Fig. 10 left) | | | OpenPose (Fig. 10 right) |
| Joint Number | Name | DOF | Reference |
| 5 | Head | 3 | - |
| 20 | Nose | 0 | a |
| 21 | Right Eye | 0 | o |
| 22 | Left Eye | 0 | P |
| 23 | Right Ear | 0 | q |
| 24 | Left Ear | 0 | r |

A link length may be obtained by another method or a link length obtained in advance may be used. Alternatively, if a skeletal model unique to the subject has been obtained, it may be used. The skeletal model unique to a subject can also be used in the motion analysis unit.

[B-4] Joint Position Obtaining Unit

The unit for obtaining joint positions is characterized in that the unit estimates position candidates for the joint position using the heatmap information obtained by the heatmap generating unit and updates the joint angles and joint positions of the skeletal model by performing optimization computation based on inverse kinematics using the candidates for the joint position. The unit for obtaining joint positions comprises a unit for obtaining candidates for joint positions estimating the candidates for the joint position based on the heatmap data, and an inverse kinematic calculation unit for calculating joint angles by performing an optimization calculation based on inverse kinematics using the joint position candidates, and a forward kinematics calculation unit calculating joint positions by performing the forward kinematics calculation using calculated joint angles.

The joint position candidate acquisition unit according to the present embodiment sets the search range for the joint position candidate using the 3D position of each joint obtained in one or a plurality of frames (for example, the joint position at a previous frame t), converts all or part of the points in the search range to the pixel positions of each camera image plane, and determine one or a plurality of jont position candidates for each joint position in a frame at time t+1 by using the spatial distribution of the likelihood obtained in a frame at time t+1.

A case where the search range is set using the joint position at the previous frame t will be described. Because of a minute change of a joint position in one frame, joint position $^{t+1}J^n$ exists in the vicinity of joint position $^tJ^n$ where a joint position of joint n in a frame at time t is $^tJ^n$ and a joint position of joint n in a frame at time t+1 is $^{t+1}J^n$. Consider $(2k+1)^3$ pieces of lattice points (k is a positive integer) distributed around $^tJ^n$ with a spacing s and a set of points is expressed as:

$$^t\mathbb{J}^n := \left\{ {}^t J^n + s \begin{bmatrix} a \\ b \\ c \end{bmatrix} \,\middle|\, -k \leq a, b, c \leq k \right\} \qquad (1)$$

k : constant positive integers
a, b, c : integers

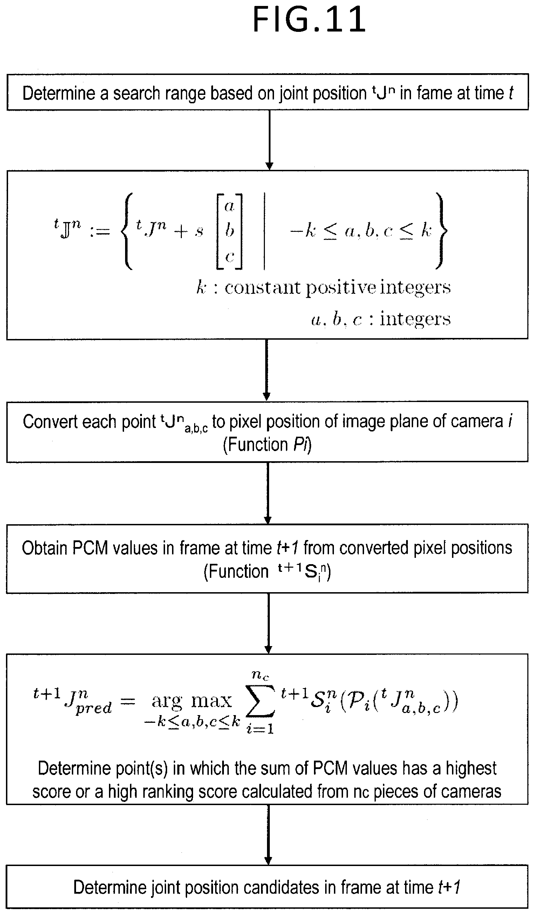

For example, as expressed in FIG. 11, consider $11 \times 11 \times 11$ points (k=5) aligned in a grid-like manner around $^tJ^n$ with an interval of s. The distance s between lattice points is independent of the size of the image pixels.
Note that all points in $^t\mathbb{J}^n$ can be converted to the pixel coordinates of the projection plane of an arbitrary camera using a function $P_i$. Supposed that a function $P_i$ denotes a function for converting one point $^tJ^n_{a,b,c}$ in $^t\mathbb{J}^n$ to a pixel position of the image plane of the camera i and a function $^{t+1}S^n_i$ denotes a function for obtaining PCM values in a frame at time t+1 from the pixel position, the maximum point of the sum of the PCM values calculated from $n_c$ pieces of cameras can be regarded as the most probable position of the joint n in the frame at time t+1, and $^{t+1}J^n_{pred}$ is obtained by

$$^{t+1}J^n_{pred} = \arg\max_{-k \leq a,b,c \leq k} \sum_{i=1}^{n_c} {}^{t+1}\mathcal{S}^n_i(\mathcal{P}_i({}^t J^n_{a,b,c})) \qquad (2)$$

This calculation is executed for $n_j$ pieces of all joints (18 pieces in the case of OpenPose).
Note that each joint position $^{t+1}J^n$ of the frame at time t+1 is a function of the joint angle $^{t+1}Q$, as expressed in the equation (3), the joint angle $^{t+1}Q$ is obtained by an optimization calculation based on the inverse kinematics, and the joint position $^{t+1}J^n$ is obtained by a forward kinematics calculation.

$$^{t+1}Q = \arg\min \sum_{n=1}^{n_j} \frac{1}{2} {}^{t+1}W^n \|{}^{t+1}J^n_{pred} - {}^{t+1}J^n({}^{t+1}Q)\|^2 \qquad (3)$$

Here, the sum of the PCM values in the prediction position of each joint is used for the weight of each joint in the optimization calculation based on the inverse kinematics $^{t+1}W^n$ as defined as:

$$^{t+1}W^n = \sum_{i=1}^{n_c} {}^{t+1}\mathcal{S}^n_i(\mathcal{P}_i({}^{t+1}J^n_{pred})) \qquad (4)$$

Each joint position obtained by the joint position obtaining unit is stored in the storage unit as time series data of the joint positions. In the embodiment, each joint position obtained by the joint position obtaining unit is smoothed by the smoothing processing unit to generate a smoothed joint position.
In the present embodiment, inverse kinematics-based optimization calculations may be performed not only by searching for the maximum value according to the equation (2), but by searching for a plurality of points (for example, seven points) among all the lattice points as a group of joint position points defining a location where the PCM score is high.
As described above, in the present embodiment, as a method of searching for a point in the 3D space having the maximum PCM score, a set of points in the 3D space is projected onto a 2D plane, and the PCM values of the pixel coordinates are acquired. Then, the sum (PCM score) of the PCM values is obtained, and the point with the highest PCM score in the set of points is set as a joint position candidate as a point in the 3D space where the PCM score is maximum. The calculation for projecting a 3D point to each camera plane and calculating the PCM score is light. According to the search for joint position candidates of the present embodiment, a reduction in calculation amount and an exclusion of outliers are realized by limiting the search range using the information of the previous frame and reprojecting the three-dimensional positions of the lattice points in the search range onto a two-dimensional image (PCM).

[B-5] Smoothing Processing Unit

Acquisition of PCM and the optimization calculation based on the inverse kinematics performed in the unit for obtaining joint positions do not consider time series relationships so that it is not guaranteed that outputted joint positions are smooth in a temporal direction. The smoothing processing unit comprises a unit for obtaining smoothed joint positions which considers temporal continuity and performs a smoothing processing using time series information of joints. For example, when smoothing the joint positions obtained in a frame at time t+1, the joint positions obtained in the frame at time t+1, the joint positions obtained in a frame at time t and the joint positions obtained in a frame at time t-1 are typically used. Regarding the joint positions obtained in the frame at time t and the joint positions obtained in the frame at time t-1, the joint positions prior to the smoothing processing for the joint position in the frame at time t+1 are used but the joint positions after the smoothing processing for the joint position in the frame at time t+1 may be used. The unit for obtaining smoothed joint positions does not necessarily use adjacent sequential frames. To simplify the calculation, the smoothing processing is performed without using body structural information, so the link length, which is a distance between adjacent joints are not maintained. Then the smoothing processing while maintaining the link length is performed by the optimization calculation based on the inverse kinematics using the skeletal structure of the subject and smoothed joint positions to obtain each joint angles of the subject.

The unit for obtaining smoothed joint positions performs a temporal smoothing processing for the joint positions by a low pass filter. The smoothing processing with a low pass filter is applied to the joint positions obtained by the unit for obtaining the joint positions, and the optimization calculation is performed based on inverse kinematics by setting a smoothed joint position as a target position of each joint. A smoothness of the temporal change of joint position can be effective in the skeletal condition where a distance between joints is a constant. If each joint position obtained by the low pass filter is used as an output of video motion capture, the temporal smoothness of each joint can be obtained, but the condition that the distance between adjacent joints is constant may be broken. In this embodiment, the joint position after the application of this low pass filter is set as the target joint position for each joint, and the optimization calculation based on inverse kinematics is performed again. Equation (3) can be used for the optimization calculation based on inverse kinematics, but the optimization calculation based on inverse kinematics is performed assuming that the weights of all joints are uniform (but not limited to this). By so doing, the smoothness of the temporal change of the joint position adapted to the low pass filter can become effective the skeletal condition that each distance between joints is invariant.

An output of the smoothing processing unit includes, for example, joint angle information and skeletal structure, and joint position information that can be uniquely calculated from the two pieces of information. For example, when drawing CG, the movement of the body is drawn by the forward kinematics calculation using the joint angle information and a file of the skeletal structure of the body. The information included in the output of the smoothing processing unit may be stored in the storage unit.

[B-6] Flow of Acquisition of Motion Data of Subject

Steps for obtaining the joint angles and the positions of the feature points from input images in accordance with the embodiment will be described. A plurality of synchronized cameras capture the motion of the subjec on the walking surface of the treadmill, and each camera outputs an RGB image at a predetermined frame rate. When the heatmap acquisition unit receives the input image, it generates a heatmap (spatial distribution of the likelihood of the joint position) for all the feature points in the body of the subject on the based on the input image.

The joint position acquisition unit searches for joint position candidates using the heatmap information of each image. In one aspect, when the heatmap generated from the input image in a frame at time t + 1 is received, the search range is set based on the joint position in a frame at time t, and the joint position candidate is searched. Then, the same process is executed for all joints, and joint position candidates for all joints are acquired.

The optimization calculation based on the inverse kinematics is performed for position candidates for all the feature points. The position candidates and the joints of the skeleton model are corresponded with each other, and the skeleton model is adapted to a skeleton model unique to a subject. Based on the candidates and weights for the positions of the feature points, the joint angles and joint positions are acquired by executing the optimization calculation based on the inverse kinematics and the forward kinematics calculation.

A smoothing processing is applied to the obtained position of feature point by using joint positions in the past frames to smooth the temporal movement of the joint position. The optimization calculation based on the inverse kinematics by using the positions of the smoothed feature points is again performed to obtain the joint angles of the subject, and joint positions are obtained by performing the forward kinematics calculation by using the obtained joint angles.

A plurality of cameras are inegrated into the frame of the treadmill so as not to install cameras and attach markers to the subject. In addition, a treadmill integrated with motion caputure can be provided in substantially the same space as the space occupied by the treadmill.

[C] Motion Analysis Unit

The motion data acquisition unit acquires time-series data of the 3D pose of the subject during walking on the walking surface. The 3D pose of the subject in each frame is defined by the three-dimensional coordinates of all the feature points (joint positions). The motion analysis unit performs various motion analysis using the time series data of the 3D pose of the subject. The motion analysis unit executes gait analysis using, for example, three-dimensional motion measurement. For example, it is possible to determine the subject stumbling in the anteroposterior direction or the lateral direction from the center of gravity position and sway data of the center of gravity. A Lissajous figure for the position of the center of gravity of the subject or the position of a certain part of the body (for example, the position of a joint) may be generated. In addition, the motion analysis unit estimates the muscle tension and muscle activity of the subject during walking, visualizes and displays them on the display.

To exemplify the information that can be acquired by motion analysis, the position of the center of gravity of the subject in each frame, the time-series data of the position of the center of gravity of the subject, the sway data of the center of gravity of the subject, the sway data of a predetermined part of the body of the subject, velocity and acceleration of the whole or part of the body of the subject, variation in joint angle of certain joints of the body of the subject, a stride length, a difference between left and right stride lengths, Lissajous figures of the position of the center of gravity of the subject and the position of a certain part of the body, and the other existing walking movements Information obtained by three-dimensional motion analysis can be mentioned. Motion analysis can be performed by using any one or a combination of a plurality of these pieces of information.

The motion analysis unit can perform motion analysis using the operation information of the treadmill in addition to the motion data acquired by the motion data acquisition unit. The operation information of the treadmill comprises, for example, the traveling speed and the incline angle of the endless belt when the subjec walks on the walking surface. The motion data of the subject and the operation data of the treadmill are stored in the storage unit in synchronization. For example, by quantifying or visualizing the movement of the whole body including the movement of the upper body such as the movement of the arm and shoulder of the subject in detail in synchronization with the treadmill, the effect of rehabilitation that appears as smooth movement of the whole body can be quantified. In addition, the swing of the center of gravity of the subject, the movement of the foot, and the movement of the treadmill can be observed in conjunction with each other. In addition, by observing the change in the movement of the whole body of the subject when the running speed of the endless belt of the treadmill is changed, it is possible to evaluate the effect of rehabilitation that cannot be observed only by the treadmill or motion capture.

When the treadmill is provided with a force plate, the motion analysis unit may perform motion analysis using the information acquired by the force plate. For example, the landing and leaving of the foot may be detected and used for motion analysis. Further, the biological information (heart rate, oxygen uptake, etc.) of the subject during walking may be acquired, and the biological information may be used in the analysis of the motion analysis unit.

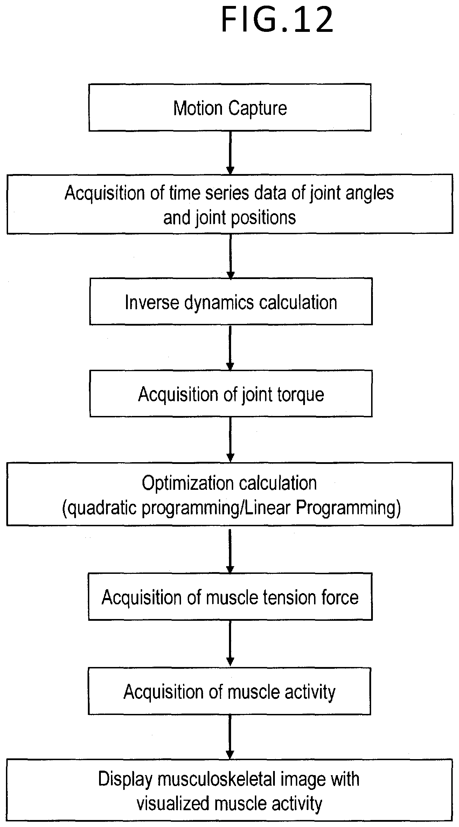

FIG. 12 illustrates an operational process of motion analysis using motion capture. The motion capture according to the present embodiment acquires time-series data of the joint angles and joint positions of the subject during walking. Further, based on the time series data, the joint torque is acquired by inverse dynamics calculation, and a wire tension in the musculoskeletal model equipped with the wires that imitate the muscles is obtained by performing an optimization calculation (a quadratic programming method or linear programming) using the joint torque. A muscle activity is calculated by using the wire tension, a musculoskeletal image with a color assigned according to the level of muscle activity is generated, and a musculoskeletal image with visualized muscle activity is outputted to a display at a predetermined frame rate to display as a motion video on the display. The details will be described below.

By interpolating a temporal displacement between frames for of all degrees of freedom of the skeleton acquired by motion capture with a continuous function, the displacement of all degrees of freedom of the skeleton at each frame time, a velocity as a time derivative of the displacement, and acceleration as a time derivative of velocity are calculated. A position, angle, velocity, angular velocity, acceleration, and angular acceleration of each link calculated from them are sent to an inverse dynamics engine, and joint torques that match the motion are calculated by calculating mechanics information according to a motion of a skeleton with assumed masses. Each segment of the skeleton is a rigid body, and its mass, center of gravity position, and inertia tensors can be estimated from statistical measurement information of each part of a person using physique information. Alternatively, these parameters can be estimated by identification from the motion information of a subject. The physique information of the subject used for the estimation may be acquired in advance.

Tension forces of the wires distributed throughout the body, which model the muscles, are calculated using the biased and weighted quadratic programming. Non-Patent Document 4 can be referred to for the calculation of the wire tension. Measurement values of the force distribution when the antagonist muscles are used according to classified motions are obtained, and biases and weights with reference to the measurement values are used to obtain a solution that better approximates the actual muscle tension. An electromyogram or a force plate may be used in obtaining the muscle tensions.

A value obtained by dividing the acquired muscle tension by the assumed maximum muscle tension is used as a muscle activity, and an image of the whole body musculoskeletal system in which the muscle color is changed according to the muscle activity is generated, and is outputted as a certain frame rate (typically 30 FPS) to the display to display as a video. An image of skeletal pose is also generated and outputted to the display at a certain frame rate to display as a video. Further, changes in the values of each variable (for example, a joint angle, a velocity, a muscle tension, a floor reaction force, a center of gravity, etc.) are graphed and outputted. These outputs may be presented as analysis results by images and graphs and are used as records of muscle and body activities or motions of each part of the body during exercise. In this way, steps of taking images of a motion of a subject, obtaining three-dimensional pose of the subject during the motion, and estimating and visualizing the muscle activity required for the motion can automatically and efficiently be performed.

As described above, the motion analysis unit reproduces the time series information of the 3D pose of the skeleton of the subject walking or running on the walking surface of the treadmill. In addition, muscle tension and muscle activity during exercise can be estimated by performing kinetic calculations that take mass into consideration. By equipping a treadmill with a motion capture ability and a motion analysis ability, it is possible to complete what used to require an elaborate measurement environment with just the treadmill. In addition, until now, respective engineers for a treadmill, motion capture, motion analysis and such have collaborated to perform motion analysis, but since these can be performed altogether, it leads to labor saving and time saving. By using the marker-less motion capture according to the present embodiment, the burden on both the subject wearing the marker and a physiotherapist is greatly reduced, and it is possible to evaluate and analyze the motion of the subject, and visualize the results as appropriate with almost the same preparation as a normal treadmill.

[D] Treadmill Operation Control Unit

As shown in FIG. 7, the treadmill according to the present embodiment comprises a control unit for controlling the operation of the treadmill based on the motion data (typically, time-series data of the pose of the subject during walking) obtained by the motion data acquisition unit, and/or, the analysis data acquired by the motion analysis unit.

In the present specification, the motion data includes a pose of the subject, time-series data of the pose of the subject, and information obtained based on the pose of the subject and/or the time-series data of the pose. The latter information does not prevent duplication with the information obtained by motion analysis. The motion data may be, for example, position information of the whole body or a part of the body of the subject (specified by one or more joint positions) in a certain frame. The information that can be acquired by motion analysis comprises, for example, the position of the center of gravity of the subject in each frame, the time-series data of the position of the center of gravity of the subject, the sway data of the center of gravity of the subject, the sway data of a predetermined part of the body of the subject, velocity and acceleration of the whole body or part of the body of the subject, variation in joint angle of a given joint of the body of the subject, a stride length, a difference between left and right stride lengths.

The operation of the treadmill comprises the rotation of the endless belt and the incline of the endless belt. The control of the rotation of the endless belt is to control the rotation speed (belt speed) via the drive unit (motor), and comprises deceleration and increase of the belt speed, maintainning of a speed of belt, stop of the belt after deceleration, stop of the belt, and reverse rotation of the endless belt. Further, the rotation speeds of the two endless belts traveling in parallel may be controlled independently.

The incline of the endless belt comprises, for example, the incline of the tread base that supports the upper part of the endless belt and the endless belt via the drive unit (motor). The change in incline of the belt comprises the increase of an incline angle of the endless belt (the walking surface may become an uphill or a downhill), reduction of an incline angle (movement from the inclined state to the flat state), and maintaining of an incline angle (including the flat state). The incline of the endless belt may include a lateral incline in addition to a vertical incline.

The control unit that controls the operation of the treadmill will be described in detail with reference to FIG. 7A. The control unit comprises an input data generation unit that generates input data based on motion data acquired by motion capture, and a control signal generation unit that generates a control signal according to the input data in which the control unit ouputs the control signal to the drive unit to obtain the desired belt speed and inclination. The control by the control unit is executed according to one or more control programs stored in the memory. The control program defines how and what kind of information is used to execute what kind of control. The input data generation unit generates input data according to the control program. The control signal generation unit generates a control signal according to the input data according to the control program.

In one embodiment, the control unit controls the operation of the treadmill when an abnormal state or anomaly (some accident has occurred in the moving object) of the subject is detected based on the motion data. The control program defines the information (input data) used for abnormality detection and the conditions for determining the abnormality. The control program, for example, detects a change from the normal state (specified based on the motion data) of the subject to an abnormal state (specified based on the motion data) or the continuation of the abnormal state, and determines an abnormality. Abnormalities in the subject can be detected, for example, by comparing the pose of the subject, changes in the pose of the subject, and changes in the center of gravity (fluctuations can be detected from changes in the pose and changes in the center of gravity) with predetermined reference values or threshold values. The operation control of the treadmill when an abnormality is detected is typically a deceleration of the belt speed or a stop after the deceleration.

In one embodiment, the control unit slows down the belt speed or stops the belt after decelation when the subject is too close to the front or rear end of the walking surface. For example, based on the position information of the whole body or a predetermined part of the subject detected based on the motion data, it is detected that the subject deviates rearward (y-axis direction) from a normal position (a predetermined area on the walking surface), and the belt speed may be reduced slowly. Alternatively, the belt speed may be slowly reduced by detecting that the position of the center of gravity of the subject deviates rearward (y-axis direction) from the normal position (a predetermined area on the walking surface).

For example, based on the position information of the whole body or a predetermined part of the subject detected based on the motion data, it is detected that the subject deviates forward (y-axis direction) from the normal position (a predetermined area on the walking surface), and the belt speed may be increased slowly. Alternatively, the belt speed may be slowly increased by detecting that the position of the center of gravity of the subject deviates forward (y-axis direction) from the normal position (a predetermined area on the walking surface).

After decelerating the belt speed as described above, the belt speed may be increased to return to the original speed upon detecting that the subject has returned to the normal position. Similarly, after increasing the belt speed, the belt speed may be reduced to return to the original speed upon detecting that the subject has returned to the normal position.

The control unit further detects that the subject deviates laterally (x-axis direction) from the normal position (a predetermined area on the walking surface) based on the position information of the whole body or a predetermined part of the target detected based on the motion data, and reduce the belt speed slowly or stop the belt after deceleration. Alternatively, the belt speed may be slowly reduced or the belt may be stopped after decelation upon detecting that the position of the center of gravity of the subject deviates from the normal position (a predetermined area on the walking surface) in the lateral direction (x-axis direction). Alternatively, the belt speed may be slowly decelerated or the belt may be stopped after decelation upon detecting the magnitude of the acceleration information or the magnitude of the sway of the center of gravity of a part of the subject.

Further, when the belt is in the uphill posture, the control unit may reset the incline of the belt to slowly return to a flat state, and/or reduce the belt speed slowly or stop the belt after decelaration upon detecting that the subject is positioned rearward from the normal position (a predetermined area on the walking surface) based on the position information of the whole body or the predetermined part of the subject detected based on the motion data.

In a dual-belt treadmill, the traveling speed of either or both belts may be adjusted. The operation control of the dual-belt treadmill will be described with reference to FIG. 7B. The dual-belt treadmill comprises two belts that can be operated independently by each belt drive unit, an incline drive unit that changes the incline of the belt, and an operation unit in which the belt drive unit and the incline drive unit operate in accordance with instructions from the operation unit. The incline direction of the belt by the incline drive unit is typically a longitudinal incline (the heights of the front and rear ends of the belt are different), but in addition to or instead of the longitudinal inlcine, the incline direction may be a lateral incline (the heights of the left and right ends of the belt are different). The dual-belt treadmill is used, for example, as a gait analysis system, a gait training system, or a gait rehabilitation support system for hemiplegic patients. Although FIG. 7B shows a dual-belt treadmill with marker-less motion capture, the technical idea of controlling the operation of the treadmill according to the motion data of the subject acquired by the motion capture is not limited to the marker-less motion capture and the dual-belt treadmill.

The treadmill comprises a control unit that controls the operation of the treadmill according to the motion data of the subject acquired in real time by motion capture. The motion data of the subject walking on the two belts is acquired by marker-less motion capture. The motion data is typically time-series data of the 3D pose of the subject, and from the time-series data of the 3D pose, for example, the 3D position of the whole body, the 3D position of the part, the pose of the whole body, the pose of the part, motion of the whole body, motion of the part (limb, foot, etc.), velocity of all or part of the body of the subject, position of the center of gravity, sway of the center of gravity, and sway of the part can be obtained. It will be understood by those skilled in the art that these data are examples of data that can be used in the control unit, and that various information can be generated based on the time series data of the 3D pose depending on matters to be controlled. The control unit controls the operation of the treadmill according to the predetermined control program in response to the input of the data generated according to the control program.

In one embodiment, the control unit independently controls the traveling speed of each belt according to the motion data. For example, the speed of one of the belts may be adjusted according to the velocity of movement of the left and right legs and the difference in stride length between the left and right legs. In conventional walking rehabilitation for hemiplegic patients, the current situation is that a physiotherapist changes the speed of the running belt on the paralyzed side while observing the condition of the patient's paralyzed leg. In the dual-belt treadmill according to the present embodiment, the speed change (accelerating or decelerating) of the traveling belt according to the movement of the paralyzed leg can be automatically controlled by the control unit. Also, in response to the motion of the patient on the belt in which the body of the patient is leaning forward and the patient is falling down, the running belt may be stopped or the belt may be appropriately accelerated to correct the forward leaning state and lead to correct walking.

In one embodiment, the control unit controls the incline of both belts according to the motion data. For example, upon detecting a pose indicating that the patient has bent forward on the belt, in accordance with the pose, the incline drive unit may be controlled to adjust the incline of the belt.

The speed control by the control unit may include reverse rotation of the endless belt in addition to acceleration, deceleration, and stop of the belt. For example, for a patient who has difficulty in stepping forward on a paralyzed leg, the traveling belt may be rotated in the reverse direction when the paralyzed leg is stepped forward to support walking.

When the treadmill (whether single belt type or dual-belt) is provided with a movable portion (for example, operated by a motor), the operation of the movable portion may be controlled. For example, the handrail bar may be moved in the front-rear direction, and the handrail bar may be moved according to the motion of the hand or arm of the subject.

[E] Walking Rehabilitation Support System

For supporting walking rehabilitation, a method of controlling the belt speed of the treadmill using the walking data of the patient (subject) on the treadmill will be described.

A first control method is to drive the belt at a speed that matches the patient's athletic performance. If the walking speed is too slow for the patient's athletic ability, the effect of rehabilitation diminishes, and conversely, if the patient tries to walk at high speed forcibly, there is a risk of falling. The belt spped control according to this embodiment allows to output the belt speed as fast as possible within the range in which the patient can walk safely and comfortably. A second control method is to allow the patient to maintain an appropriate walking pose close to upright even if the patient has a difference in athletic ability between the left and right legs. If there is a difference in the athletic ability of both legs due to hemiplegia or the like, the person drags the leg with the lower athletic ability when walking. By making a difference in the left and right belt speeds of the treadmill, the difference in athletic ability of both legs is compensated. In this embodiment, a belt speed control method that combines the first control method and the second control method is provided.

The first control method will be described. As the first control method, two kinds of controls, position control and speed control are proposed. Position control will be described. If the belt speed is slow and the patient can walk comfortably, the patient moves forward on the treadmill. On the other hand, if the belt speed is high and the patient's walking speed cannot keep up, the moving belt pushes the patient back. A method is adopted in which the belt speed is matched to the walking speed of the patient by maintaining the walking position of the patient at a predetermined position (typically near the center) on the walking surface of the treadmill. As an index showing the walking position of the patient, the three-dimensional position estimation results of the left and right hip joints are output from the video motion capture. In the present embodiment, the midpoint of the left and right hip joints is regarded as the "lumbar center", and the lumbar center is regarded as the pseudo body center. The y-coordinate of the lumbar center at time t can be obtained from the y-coordinate $y_{Lplv}(t)$ of the left hip joint and the y-coordinate $y_{Rplv}(t)$ of the right hip joint at time t. In the present embodiment, the difference between the y-coordinate average value of the lumbar center and the target value $y^{ref}_{plv}$ of the lumbar center during one walking cycle (for example, 1 second) $\Delta y_{plv}(t)$

$$\Delta y_{plv}(t) \;=\; \bar{y}_{plv}(t) - y^{ref}_{plv}$$

is controlled to approach zero. The target value $y^{ref}_{plv}$ is set according to the coordinate system set at the time of calibration, and in this embodiment, $y^{ref}_{plv}=0$ mm.

Speed control will be explained. When the belt speed and the patient's walking speed are perfectly balanced, the patient's walking position is kept constant on the treadmill. That is, the moving velocity of the patient in the world coordinate system indicates a value close to zero. The belt speed is adjusted so that the patient's velocity in the world coordinate system is zero. At time t, the average value of the velocity v $_{plv, y}$(t) in the y direction of the lumbar center in the world coordinate system over the past c seconds is expressed as follows.

$$\bar{v}_{plv,y}(t) \;\; = \;\; \frac{1}{c}\big(y_{plv}(t) - y_{plv}(t - cf)\big)$$

Control is performed so that this value approaches zero.

The second control method will be described. When there is a difference in athletic ability between the patient's left and right legs, which causes the patient to be in a walking pose in which the patient drags one leg, and a difference between the motion ranges of both legs of the patient is occurred in an anteroposterior direction (y-axis direction). This is directly due to the difference in stride length between the left and right legs. By making a difference between the left and right belt speeds of the treadmill, the difference in the motion abilities of both legs is compensated to align the centers of the motion ranges of both legs. Assuming that the y-coordinates of the patient's left ankle and right ankle are detected as $y_{L, ank}$(t) and $y_{R, ank}$(t) in the video motion capture result at time t, the centers of the motion ranges of the left leg and right leg with respect to the y direction are obtained respectively as follows.

$$\bar{y}_{L,ank}(t) \;\; = \;\; \frac{1}{2}\big(\max_{t-f+1 \le T \le t} y_{L,ank}(T) + \min_{t-f+1 \le T \le t} y_{L,ank}(T)\big)$$

$$\bar{y}_{R,ank}(t) \;\; = \;\; \frac{1}{2}\big(\max_{t-f+1 \le T \le t} y_{R,ank}(T) + \min_{t-f+1 \le T \le t} y_{R,ank}(T)\big)$$

Here, $\Delta y_{LRa}$(t) is defined as follows.

$$\Delta y_{LRa}(t) \;\; = \;\; \bar{y}_{L,ank}(t) - \bar{y}_{R,ank}(t)$$

Since this $\Delta y_{LRa}$(t) represents a difference of the centers of the ranges of motion of both legs in the y direction, the second control method that brings $\Delta y_{LRa}$(t) closer to zero is added to the position control or speed control in the first control method. That is, the second control method can be applied in combination with the position control or speed control of the first control method. The first control method (position control or speed control) can also be used for the exercise of an able-bodied person or an athlete.

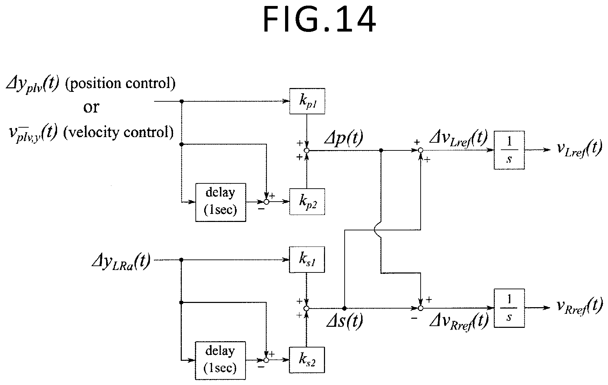

The calculation method of the target belt speed will be described. FIG. 14 shows a block diagram showing a method of calculating the left belt target speed $v_{L, ref}$(t) and the right belt target speed $v_{R, ref}$(t) of the treadmill at time t. First, the initial velocities $v_{L, ref}$(0) and $v_{R, ref}$(0) of both belts are determined as follows.

$$v_{L,ref}(0) = v_{R,ref}(0) = 3.0\,\mathrm{km/h}$$

In the present embodiment, the belt speeds $v_{L, ref}$(nf) and $v_{R, ref}$(nf) at the time nf seconds are updated for each f frame according to the following update formula using the motion data of the subject on the treadmill.

$$v_{L,ref}((n+1)f) = v_{L,ref}(nf) + \Delta v_{L,ref}(nf)$$

$$v_{R,ref}((n+1)f) = v_{R,ref}(nf) + \Delta v_{R,ref}(nf)$$

Here, $\Delta v_{L, ref}$(nf) and $\Delta v_{R, ref}$(nf) are correction terms for updating the belt speed.

The calculation method of $\Delta v_{L, ref}$(nf) and $\Delta v_{R, ref}$(nf) will be described. First, e (t) is defined as follows.

$$e(t) = \begin{cases} \Delta y_{plv}(t) & (\text{Position Control}) \\ \bar{v}_{plv,y}(t) & (\text{Speed Control}) \end{cases}$$

Using two constants $k_{p1}$ and $k_{p2}$, $\Delta p(t)$ is defined as follows.

$$\Delta p(t) = k_{p1}e(t) + k_{p2}(e(t) - e(t - f + 1))$$

Using two constants $k_{s1}$ and $k_{s2}$, $\Delta s$ (t) is defined as follows.

$$\Delta s(t) = k_{s1}\Delta y_{LRa}(t) + k_{s2}(\Delta y_{LRa}(t) - \Delta y_{LRa}(t - f + 1))$$

Using $\Delta p$ (t) and $\Delta s$ (t), $\Delta v_{L, ref}$ (nf) and $\Delta v_{R, ref}$ (nf) are defined as follows.

$$\Delta v_{L,ref}(nf) = \Delta p(nf) + \Delta s(nf)$$

$$\Delta v_{R,ref}(nf) = \Delta p(nf) - \Delta s(nf)$$

The values of $k_{p1}$, $k_{p2}$, $k_{s1}$, and $k_{s2}$ are appropriately set by those skilled in the art. For example, $k_{p1}$ = 1.0, $k_{p2}$ = 0.5, $k_{s1}$ = 0.3, $k_{s2}$ = 0.1. As shown in FIG. 14, in the present embodiment, by combining the treadmill with marker-less motion capture, with only one means, namely, motion capture, a plurality of controls according to the first control method and the second control method can be performed at the same time based on the motion data acquired by the motion capture.

**Claims**

1. A treadmill integrated with motion capture comprising:

    a frame;
    an endless belt that provides a walking surface on which a subject stands;
    a belt drive unit;
    a plurarity of cameras provided on the frame in which orientations and/or locations of cameras are adjustable; and
    a motion data acquisition unit that acquires motion data of the subject markerlessly using image information obtained from each camera image.

2. The treadmill according to claim 1 wherein the motion data includes a pose of the subject and time-series data of the pose.

3. The treadmill according to claim 2 wherein the motion data includes information obtained based on the pose of the subject and/or the time-series data of the pose.

4. The treadmill according to any one of claims 1 and 2 wherein the pose of the subject is obtained as three-dimensional coordinate values defined by a first axis in the traveling direction of the endless belt, a second axis in the widthwise direction of the endless belt, and a third axis in the vertical direction.

5. The treadmill according to any one of claims 1 to 4 wherein the motion data acquisition unit acquires a spatial distribution of the likelihood of the joint position of the subject by using the image information of the subject obtained by each camera image, and acquires a three-dimensional pose of the subject in each frame based on the spatial distribution of the likelihood.

6. The treadmill according to any one of claims 1 to 5, further comprising a motion analysis unit that analyzes a motion of the subject using the motion data.

7. The treadmill according to claim 6 wherein the motion analysis unit analyzes the motion of the subject by using the operation information of the endless belt in addition to the motion data.

8. The treadmill according to any one of claims 1 to 7, further comprising a control unit that controls the operation of the treadmill based on the motion data.

9. The treadmill according to claim 8 wherein the control unit comprises:

a data generation unit that generates input data based on the motion data; and
a control signal generator that generates a control signal according to the input data, and wherein the control unit outputs the control signal to the belt drive unit to control the belt speed.

10. The treadmill according to claim 9 wherein the endless belt is inclinable by an incline drive unit, wherein said control unit outputs the control signal to the incline drive unit to control the incline of the belt.

11. The treadmill according to any one of claims 9 and 10 wherein the control unit controls the belt speed according to the position information of the subject obtained based on the pose of the subject.

12. The treadmill according to claim 11 wherein the control unit controls the belt speed so that the position of the predetermined portion of the subject corresponds to a target value.

13. The treadmill according to any one of claims 9 and 10 wherein the control unit controls the belt speed according to the velocity of a predetermined part of the subject obtained based on the time-series data of the pose of the subject.

14. The treadmill according to claim 13 wherein the control unit controls the belt speed so that the moving velocity of the predetermined portion of the subject in the world coordinate system approaches zero.

15. The treadmill according to any one of claims 8 to 14 wherein the endless belt comprises a first endless belt on which the left foot of the subject rests and a second endless belt on which the right foot of the subject rests, said drive unit comprises a first drive unit for the first endless belt and a second drive unit for the second endless belt, and said control unit independently controls each of the speed of the first endless belt and the speed of the second endless belt according to the motion data.

16. The treadmill according to claim 15 wherein, when there is a difference between the left and right stride lengths obtained based on the motion data, the control unit controls the speed of the first endless belt and the speed of the second endless belt so as to compensate for the difference in stride length.

17. The treadmill according to any one of claims 8 to 10 wherein the control unit controls the operation of the treadmill when an abnormality of the subject is detected based on the motion data.

18. The treadmill according to claim 1 wherein a calibration device can be installed on the treadmill, said calibration device comprising:

a checkerboard located vertically in the middle of the endless belt in the front-rear direction;
on the first surface of the checkerboard, a first checker pattern that can be photographed by one or more of the plurality of cameras being provided; and
on the second surface of the checkerboard, a second checker pattern that can be photographed by the remaining one or more of the plurality of cameras being provided.

19. A treadmill control system comprising:

an endless belt;
a belt drive
marker-less motion capture that captures the motion of a subject on said endless belt; and
a control unit that controls the operation of the treadmill,

wherein the control unit controls the operation of the treadmill according to motion data of the subject acquired by the motion capture.

20. The treadmill control system according to claim 19 wherein said control unit comprises: a data generation unit that generates input data based on the motion data; and a control signal generator that generates a control signal according to the input data, wherein said control unit outputs the control signal to the belt drive unit to control the speed of the belt.

21. The treadmill control system according to claim 20 wherein the endless belt is inclinable by an incline drive unit, and the control unit outputs the control signal to the incline drive unit to control the incline of the belt.

22. The treadmill control system according to any one of claims 20 and 21 wherein the control unit controls the belt speed according to the position information of the subject obtained based on the pose of the subject.

23. The treadmill control system according to claim 22 wherein the control unit controls the belt speed so that the position of the predetermined portion of the subject matches the target value.

24. The treadmill control system according to any one of claims 20 and 21 wherein the control unit controls the belt speed according to the speed of a predetermined part of the subject obtained based on the time-series data of the pose of the subject.

25. The treadmill according to claim 24 wherein the control unit controls the belt speed so that the moving speed of the predetermined portion of the subject in the world coordinate system approaches zero.

26. The treadmill control system according to any one of claims 19 to 25 wherein the endless belt comprises a first endless belt on which the left foot of the subject rests and a second endless belt on which the right foot of the subject rests, said drive unit comprises a first drive unit for the first endless belt and a second drive unit for the second endless belt, and said control unit independently controls each of the speed of the first endless belt and the speed of the second endless belt according to the motion data.

27. The treadmill control system according to claim 26 wherein, when there is a difference between the left and right stride lengths obtained based on the motion data, the control unit controls the speed of the first endless belt and the speed of the second endless belt so as to compensate for the difference in stride length.

28. The treadmill control system according to any one of claims 19 to 21 wherein the control unit controls the operation of the treadmill when an abnormality of the subject is detected based on the motion data.

# FIG.1

C1

C2

9

80

8

80

5

1

7

2

5

7

3

4A

4B

2

5

4

2

6

6

C3

# FIG.1A

# FIG.2

# FIG.3

# FIG.4

# FIG.5

# FIG.6

# FIG.7

# FIG.7A

```
┌─────────────────────────────────────────────┐
│                Motion Capture                 │
└─────────────────────────────────────────────┘
                        │
                        ▼
┌─────────────────────────────────────────────┐
│          Time-series Data of 3D Pose          │
│  (3D coordinates of Joints) of Subject on Belt│
└─────────────────────────────────────────────┘
                        │
                        ▼
┌───────────────────────────────────────────────────────┐
│                  ┌───────────────────────┐  ┌─────────┐│
│                  │  Input Data Generator  │◄─►│         ││
│  Control         └───────────────────────┘   │ Control ││
│  Unit                      │                   │ Program ││
│                  ┌──────────────┐             │         ││
│                  │  Input Data  │             │         ││
│                  └──────────────┘             │         ││
│                            │                   │         ││
│                  ┌───────────────────────┐   │         ││
│                  │ Control Signal Generator│◄─►│         ││
│                  └───────────────────────┘   └─────────┘│
└───────────────────────────────────────────────────────┘
                        │
                        ▼
              ┌──────────────────┐
              │  Control Signal   │
              └──────────────────┘
                        │
                        ▼
┌─────────────────────────────────────────────┐
│         Belt Drive Unit/Tilt Drive Unit       │
└─────────────────────────────────────────────┘
                        │
                        ▼
┌─────────────────────────────────────────────┐
│               Belt Speed/Tilt                 │
└─────────────────────────────────────────────┘
```

# FIG.7B

# FIG.8

# FIG.9

Camera Calibration

Obtain intrinsic parameters and extrinsic parameters
Correct distortion

Obtain function (matrix) *Pi* for converting arbitrary point in 3D space to pixel position on projected plane of each camera

Obtain initial heatmap from each camera image

Estimate initial joint positions (Estimation of initial pose of skeletal model)

Correspondence between each joint (position estimated) and each joint of skeletal model

Obtain each distance between Joints for each subject

Update skeletal model for each subject

Various parameters, etc.

Time series data of joint positions

Skeletal structure of body

Storage Unit

# FIG.10

# FIG.11

Determine a search range based on joint position $^t\mathsf{J}^n$ in fame at time $t$

$$^t\mathbb{J}^n := \left\{ {}^t J^n + s \begin{bmatrix} a \\ b \\ c \end{bmatrix} \;\middle|\; -k \le a, b, c \le k \right\}$$

$k$ : constant positive integers

$a, b, c$ : integers

Convert each point $^t\mathsf{J}^n{}_{a,b,c}$ to pixel position of image plane of camera $i$
(Function $Pi$)

Obtain PCM values in frame at time $t+1$ from converted pixel positions
(Function $^{t+1}\mathsf{S}_i^n$)

$$^{t+1}J^n_{pred} = \underset{-k \le a,b,c \le k}{\arg\max} \sum_{i=1}^{n_c} {}^{t+1}\mathcal{S}_i^n\left(\mathcal{P}_i\left({}^t J^n_{a,b,c}\right)\right)$$

Determine point(s) in which the sum of PCM values has a highest
score or a high ranking score calculated from nc pieces of cameras

Determine joint position candidates in frame at time $t+1$

# FIG.12

```
┌─────────────────────────────────┐
│         Motion Capture          │
└─────────────────────────────────┘
                 │
                 ▼
┌─────────────────────────────────────────┐
│  Acquisition of time series data of joint angles │
│           and joint positions           │
└─────────────────────────────────────────┘
                 │
                 ▼
┌─────────────────────────────────┐
│    Inverse dynamics calculation  │
└─────────────────────────────────┘
                 │
                 ▼
┌─────────────────────────────────┐
│     Acquisition of joint torque  │
└─────────────────────────────────┘
                 │
                 ▼
┌─────────────────────────────────────────┐
│        Optimization calculation          │
│ (quadratic programming/Linear Programming) │
└─────────────────────────────────────────┘
                 │
                 ▼
┌─────────────────────────────────┐
│  Acquisition of muscle tension force │
└─────────────────────────────────┘
                 │
                 ▼
┌─────────────────────────────────┐
│    Acquisition of muscle activity │
└─────────────────────────────────┘
                 │
                 ▼
┌─────────────────────────────────────────┐
│      Display musculoskeletal image with   │
│        visualized muscle activity        │
└─────────────────────────────────────────┘
```

# FIG.13

double-face
checkerboard $^{chk2}R_{chk1}$

$O_{chk1}$

$O_{chk2}$

$^{chk1}R_{floor}$

$O_{floor}$

double-face
checkerboard

# FIG.14

$\Delta y_{plv}(t)$ (position control)
or
$v^-_{plv,y}(t)$ (velocity control)

$k_{p1}$

$+$ $\Delta p(t)$ $+$ $\Delta v_{Lref}(t)$ $\frac{1}{s}$ $\to$ $v_{Lref}(t)$
$+$ $+$

delay (1sec) $+$ $k_{p2}$
$-$

$\Delta y_{LRa}(t)$ $k_{s1}$

$+$ $+$ $\Delta v_{Rref}(t)$ $\frac{1}{s}$ $\to$ $v_{Rref}(t)$
$+$ $\Delta s(t)$ $-$

delay (1sec) $+$ $k_{s2}$
$-$

## INTERNATIONAL SEARCH REPORT

International application No.

PCT/JP2020/048346

### A. CLASSIFICATION OF SUBJECT MATTER

A63B 22/02(2006.01)i; A63B 22/06(2006.01)n; A63B 23/04(2006.01)n; A63B 24/00(2006.01)i; A63B 69/00(2006.01)i; A63B 71/06(2006.01)i
FI:     A63B22/02; A63B24/00; A63B69/00 A; A63B69/00 C; A63B71/06 M; A63B22/06 M; A63B23/04 N

According to International Patent Classification (IPC) or to both national classification and IPC

### B. FIELDS SEARCHED

Minimum documentation searched (classification system followed by classification symbols)
A63B22/00-24/00; A63B69/00; A63B71/06

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 6573739 B1 (UMEYAMA, Ko, UMEYAMA, Mitsuhiro) 11 September 2019 (2019-09-11) claims 1, 4-6, 16-17, 19-20, paragraphs [0042], [0047], [0055]-[0060], [0065]-[0071], [0091]-[0107], [0117]-[0173], [0252] | 1-28 |
| Y | claims 1, 4-6, 16-17, 19-20, paragraphs [0042], [0047], [0055]-[0060], [0065]-[0071], [0091]-[0107], [0117]-[0173], [0252] | 2-18, 20-28 |
| Y | claims 1, 4-6, 16-17, 19-20, paragraphs [0042], [0047], [0055]-[0060], [0065]-[0071], [0091]-[0107], [0117]-[0173], [0252] | 1-28 |
| Y | US 9526451 B1 (BERTEC CORPORATION) 27 December 2016 (2016-12-27) abstract, claims, column 1, lines 37-41, column 2, line 63 to column 3, line 24, column 39, line 21 to column 45, line 60, column 60, lines 24-40, column 63, lines 31-38, column 65, lines 45-51, fig. 32 | 1-28 |

☒ Further documents are listed in the continuation of Box C.   ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 March 2021 (12.03.2021) | 30 March 2021 (30.03.2021) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2020/048346 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | US 9916011 B1 (BERTEC CORPORATION) 13 March 2018 (2018-03-13) abstract, claims, column 15, lines 25-53, column 14, line 6 to column 21, line 16 | 1-28 |
| A | US 10413230 B1 (BERTEC CORPORATION) 17 September 2019 (2019-09-17) column 47, line 18 to column 48, line 13 | 1-28 |
| A | CN 109646876 A (EFEI INST PHYSYCAL SCI CAS) 19 April 2019 (2019-04-19) abstract, claims 1-2, 7, 9, paragraphs [0041]-[0065] | 1-28 |
| A | CN 106540407 A (TANG, Xiaoshi) 29 March 2017 (2017-03-29) paragraph [0043], abstract, claims, paragraphs [0001]-[0006], [0018], [0041]-[0077], [0081]-[0082] | 1-28 |
| A | KR 10-2012-0097119 A (KOREA ADVANCED INST SCI & TECH) 03 September 2012 (2012-09-03) abstract, claims, paragraphs [0001]-[0009], [0032]-[0033], [0044]-[0045], [0059], [0063] | 1-28 |
| A | KR 10-2010-0026691 A (KANG, Seong-Sik) 10 March 2010 (2010-03-10) abstract, claims, paragraphs [0001]-[0008], [0011], [0013], [0017], [0046]-[0048], [0055] | 1-28 |
| A | JP 2017-64120 A (RICOH CO., LTD.) 06 April 2017 (2017-04-06) abstract, claims, paragraphs [0001]-[0007], [0016]-[0017], [0022], [0031]-[0034], [0085]-[0086] | 1-28 |
| A | MOTIONMETRIX, TECHNOLOGY, MOTIONMETRIX AB [online], 25 December 2019 [retrieved on 12 March 2021, Retrieved from the Internet:<URL:https://web.archive.org/web/20191225 050822/http://www.motionmetrix.se/4-technology/>, in particular, columns "Markeless Motion Capture", "Biomechanical assessment", "Measurement setup", "Validation" | 1-28 |
| A | MUNRMANN, Lars et al., "Most favorable camera configuration for a shape-from-silhouette markerless motion capture system for biomechanical analysis", Proceedings Volume 5665, Videometrics VIII, PROCEEDINGS OF SPIE, SPIE Digital Library [online], 17 January 2005 [retrieved on 12 March 2021], Retrieved from the Internet:<URL:https://www.spiedigitallibrary.org/c onference-proceedings-of-spie/5665/56650T/Most-favorable-camera-configuration-for-a-shape-from-silhouette-markerless/10.1117/12.587970.full?SSO=1><DOI:10.11 17/12.587970>, abstract | 1-28 |

Form PCT/ISA/210 (continuation of second sheet) (January 2015)

**INTERNATIONAL SEARCH REPORT**
Information on patent family members

| International application No. |
| --- |
| PCT/JP2020/048346 |

| Patent Documents referred in the Report | Publication Date | Patent Family | Publication Date |
| --- | --- | --- | --- |
| JP 6573739 B1 | 11 Sep. 2019 | (Family: none) | |
| US 9526451 B1 | 27 Dec. 2016 | US 9168420 B1 | |
| US 9916011 B1 | 13 Mar. 2018 | US 10216262 B1 | |
| US 10413230 B1 | 17 Sep. 2019 | US 8847989 B1 | |
| CN 109646876 A | 19 Apr. 2019 | (Family: none) | |
| CN 106540407 A | 29 Mar. 2017 | (Family: none) | |
| KR 10-2012-0097119 A | 03 Sep. 2012 | (Family: none) | |
| KR 10-2010-0026691 A | 10 Mar. 2010 | (Family: none) | |
| JP 2017-64120 A | 06 Apr. 2017 | KR 10-2017-0038722 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4830021 A
- JP 20132226340 A
- JP 2015107247 A
- US 4601065 A
- US 8002672 B
- US 6173608 B
- JP 10243979 A
- US 5368532 A
- US 5800314 A

### Non-patent literature cited in the description

- **T. OHASHI ; Y. IKEGAMI ; K. YAMAMOTO ; W. TAKANO ; Y. NAKAMURA.** Video Motion Capture from the Part Confidence Maps of Multi-Camera Images by Spatiotemporal Filtering Using the Human Skeletal Model. *2018 IEEE/RSJ International Conference on Intelligent Robots and Systems (IROS), Madrid,* 2018, 4226-4231
- *Open pose, https://github.com/CMU-Perceptual-Computing-Lab/openpose*
- **Y. NAKAMURA ; K. YAMANE ; Y. FUJITA ; I. SUZUKI.** Somatosensory computation for man-machine interface from motion-capture data and musculoskeletal human model. *Trans. Rob.,* February 2005, vol. 21 (1), 58-66